(19) Europäisches Patentamt
European Patent Office
Office européen des brevets

(11) Veröffentlichungsnummer : **0 009 787**
**B2**

# (12) NEUE EUROPÄISCHE PATENTSCHRIFT

(45) Veröffentlichungstag der neuen Patentschrift : **09.07.86**

(51) Int. Cl.[4] : **C 07 C 17/14**, C 07 C 21/24, C 07 C 25/02// C07B39/00

(21) Anmeldenummer : **79103716.1**

(22) Anmeldetag : **01.10.79**

## (54) Verfahren zur Monohalogenierung von Alkylbenzolen in alpha-Stellung.

(30) Priorität : **11.10.78 DE 2844270**
**10.02.79 DE 2905081**

(43) Veröffentlichungstag der Anmeldung : **16.04.80 Patentblatt 80/08**

(45) Bekanntmachung des Hinweises auf die Patenterteilung : **13.01.82 Patentblatt 82/02**

(45) Bekanntmachung des Hinweises auf die Entscheidung über den Einspruch : **09.07.86 Patentblatt 86/28**

(84) Benannte Vertragsstaaten :
**CH DE FR GB IT NL**

(56) Entgegenhaltungen :
**DE-A- 1 051 270**
**DE-A- 1 051 270**
**DE-A- 1 668 243**
**DE-A- 2 150 955**
**DE-C- 478 084**
**Ullmanns Encyklopädie der technischen Chemie, 4. Auflage, Band 9 (1975) Seiten 525 bis 536**
**Inoue, J., Izumi, M. und Imoto, E.: Chem. Lett. 1973, 6, 571 bis 572**
**E.T. Mc Bee, H. B. Hass, G. M. Rothbrock, J.S. Newcomer, W. V. Clipp, Z. D. Welch und C. I. Gochenour, Ing. Eng. Chem., 1947, 39, Seiten 384 bis 386**
**Ullmanns Encyklopädie der technischen Chemie, 3. Auflage 5. Band (1954) Seite 455**
**Kirk-Othmer, Encyklopädia of Chemical Technologie, Band 5, 1979, Seiten 828 bis 838**
**A. Scipioni Ann. di Chim. 41 Seiten 491-498 (1951)**

(73) Patentinhaber : **BAYER AG**
**Konzernverwaltung RP Patentabteilung**
**D-5090 Leverkusen 1 Bayerwerk (DE)**

(72) Erfinder : **Schubart, Rüdiger, Dr.**
**An der Engelsfuhr 27**
**D-5060 Bergisch-Gladbach (DE)**
Erfinder : **Klauke, Erich, Dr.**
**Eichendorffweg 8**
**D-5068 Odenthal (DE)**
Erfinder : **Naumann, Klaus, Dr.**
**Wolfskaul 2**
**D-5000 Köln 80 (DE)**
Erfinder : **Fuchs, Rainer, Dr.**
**Roeberstrasse 8**
**D-5600 Wuppertal 1 (DE)**

EP 0 009 787 B2

**Beschreibung**

Die Erfindung betrifft ein Verfahren zur Monohalogenierung von Alkylbenzolen in α-Stellung.

Es ist bekannt, Alkylbenzole, beispielsweise Toluol, bei erhöhter Temperatur und bei Belichtung mit UV-Licht mit elementarem Chlor in der Seitenkette zu chlorieren. Hierbei entstehen Nebenprodukte, beispielsweise bei der Chlorierung von Toluol zu Benzylchlorid zusätzlich Benzalchlorid, Benzotrichlorid, kernchlorierte Produkte und kondensierte hochsiedende Nebenprodukte (Houben-Weyl, Methoden der organischen Chemie, Band V/3, Seite 736, Georg Thieme Verlag Stuttgart, 1962).

Es ist ferner aus DE-P 1 051 270 ein technisch durchführbares Verfahren zur Chlorierung von Toluol zu Benzalchlorid bekannt geworden. Bei diesem Verfahren wird jedoch, um die Bildung höher chlorierter Produkte zu vermeiden, nur bis zu einem Umsatz von 35 % gearbeitet.

Es wurde ein verfahren zur Monohalogenierung von Alkylbenzolen, die in α-Stellung mindestens 1 Wasserstoffatom aufweisen und gegebenenfals Wasser bis zur Sättigungskonzentration enthalten, in α-Stellung in flüssiger Phase im Temperaturbereich von etwa 80 °C bis zum Siedepunkt des Alkylbenzols in Gegenwart von UV-Strahlen enthaltendem Licht mit Chlor oder Brom bis zu einem Umsatz, daß die Konzentration des Monohalogenalkylbenzols im Reaktionsgemisch nicht mehr als etwa 33 Mol-%, bezogen auf die Molzahl aller im Reaktionsgemisch enthaltenden Stoffe, beträgt, gefunden, das dadurch gekennzeichnet ist, daß man die Monohalogenierung in einer Apparatur vornimmt, bei der aus einem Verdampfungsgefäß (g), in dem sich Alkylbenzol und nach Anlaufen der Reaktion Monohalogenalkylbenzol befinden, das Alkylbenzol über eine mit Füllkörpern gefüllte Kolonne (f), in die ein leeres, sich nach unten verjüngendes Rohr (e) eingelassen ist, und einen Kondensator (i) in einen Reaktionsraum (a), der sich zwischen Kolonne und Kondensator befindet und in den eine zur Zuführung des Halogens dienende Vorrichtung (b) eingelassen ist, deren im Reaktionsraum befindlicher Teil zu einer Vertiefung (c) ausgebildet ist eingebracht wird, das Reaktionsgemisch durch das vom Kondensator ständig in die Vertiefung (c) tropfende Alkylbenzol durch ein Loch in der Vertiefung (c) und über das leere Rohr (e) in die Kolonne überführt wird, wo die Trennung in Alkylbenzol, das über den Kondensator in den Reaktionsraum zurückgeführt wird und Monohalogenalkylbenzol, das in das Verdampfungsgefäß gelangt, durchgeführt wird.

Als Alkylbenzole, die in α-Stellung mindestens ein Wasserstoffatom aufweisen, seien beispielsweise solche der Formel

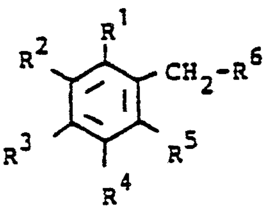

(I)

genannt, in der

$R^1$ bis $R^5$ gleich oder verschieden sein können und für Wasserstoff, Alkyl, Halogenalkyl, Aryl, Aralkyl, Alkoxy, Aryloxy, Halogen, Isocyanat, Nitro, Chlorsulfonyl, Chlorcarbonyl oder Cyano stehen, wobei außerdem zwei benachbarte Reste $R^1$ bis $R^5$ gemeinsam eine Alkylengruppe oder gemeinsam mit den sie tragenden Kohlenstoffatomen einen kondensierten aromatischen Ring bedeuten können und

$R^6$ Wasserstoff, Halogen, Alkyl oder Aryl bedeutet.

Als Alkyl seien beispielsweise geradkettige oder verzweigte Kohlenwasserstoffreste mit 1 bis 4 Kohlenstoffatomen genannt, wie Methyl, Ethyl, Propyl, Isopropyl, Butyl oder Isobutyl. Bevorzugtes Alkyl ist der Methylrest.

Als Halogenalkyl seien beispielsweise teilweise oder vollständig halogenierte geradkettige oder verzweigte Kohlenwasserstoffreste mit 1 bis 4 Kohlenstoffatomen genannt, wie Chlormethyl, Dichlormethyl, Trichlormethyl, Brommethyl, Dibrommethyl, Tribrommethyl, Monofluormethyl, Difluormethyl, Trifluormethyl, Monofluordichlormethyl, Difluormonochlormethyl, teilweise oder vollständig halogenierte Äthylreste, teilweise oder vollständig halogenierte Propylreste und teilweise oder vollständig halogenierte Butylreste. Bevorzugtes Halogenalkyl sind der Trichlormethylrest und der Trifluormethylrest.

Als Aryl seien beispielsweise aromatische Reste mit 6 bis 14 Kohlenstoffatomen genannt, wie Phenyl, Naphthyl, Anthryl oder Diphenyl. Bevorzugtes Aryl ist Phenyl.

Als Aralkyl seien beispielsweise Kohlenwasserstoffreste mit 1 bis 2 Kohlenstoffatomen im aliphatischen Teil und 6 bis 14 Kohlenstoffatomen im aromatischen Teil genannt, wie Benzyl, β-Phenyl-äthyl, Naphthyl-methyl, Naphthyl-äthyl, Anthryl-methyl, Anthryl-äthyl, ortho-, meta- und para-Diphenyl-methyl, ortho-, meta- oder para-Diphenyl-äthyl. Bevorzugtes Aralkyl ist der Benzylrest.

Als Alkoxy seien beispielsweise Reste genannt, die von einem $C_1$-$C_4$ Alkohol abgeleitet sind, wie Methoxy, Ethoxy, Propoxy, Isopropoxy, Butoxy oder Isobutyloxy, Bevorzugtes Alkoxy ist der Methoxyrest.

Als Aryloxy seien beispielsweise Reste genannt, die sich von einer phenolischen Verbindung

ableiten, wie Phenoxy, Diphenyloxy, Naphthyloxy oder Anthryloxy. Bevorzugtes Aryloxy ist der Phenoxyrest.

Als Halogen seien die Elemente Fluor, Chlor, Brom oder Jod genannt, bevorzugt Fluor, Chlor oder Brom.

Für den Fall, daß zwei benachbarte Reste $R^1$ bis $R^5$ gemeinsam eine Alkylengruppe bilden, seien Alkylengruppen mit 3 bis 4 Kohlenstoffatomen genannt, die gegebenenfalls weitere niedere Alkylreste tragen können, beispielsweise Trimethylen, methylsubstituiertes Trimethylen, Tetramethylen oder methylsubstituiertes Tetramethylen.

Weiterhin können zwei benachbarte Gruppen $R^1$ bis $R^5$ gemeinsam mit den sie tragenden Kohlenstoffatomen einen kondensierten aromatischen Ring darstellen, so daß unter Einbeziehung des aromatischen Ringes, der die Substituenten $R^1$ bis $R^5$ trägt, beispielsweise das Naphthalinsystem entsteht.

Als Ausgangsverbindungen der Formel (I) für das erfindungsgemäße Verfahren seien beispielsweise genannt : Toluol, ortho-Xylol, meta-Xylol, para-Xylol, Mesitylen, Durol, 1,2,4-Trimethylbenzol, Pentamethylbenzol, Hexamethylbenzol, ortho-Chlortoluol, meta-Chlortoluol, para-Chlortoluol, ortho-Fluortoluol, meta-Fluortoluol, para-Fluortoluol, ortho-Bromtoluol, meta-Bromtoluol, para-Bromtoluol, ortho-Cyanotoluol, meta-Cyanotoluol, para-Cyanotoluol, Dichlortoluol, Trichlortoluol, Difluortoluol, Trifluortoluol, ortho-Phenoxytoluol, meta-Phenoxytoluol, para-Phenoxytoluol, Phenoxy-chlortoluol, Phenoxyfluortoluol, ortho'- meta'- und para'-Chlorphenoxytoluol, ortho'-, meta'- und para'-Fluorphenoxytoluol, Methylnaphthalin, Methylbiphenyl, Tetrafluortoluol, Pentafluortoluol, ortho-, meta-, para-Nitrotoluol ; ortho-, meta-, para-Chlorsulfonyltoluol, Benzylchlorid, alle isomeren im Benzolkern Chlor-Fluor-substituierten, Brom-Fluor-substituierten und Jod-Fluor-substituierten Toluole.

Als Überschuß der Ausgangsstoffe der Formel (I) gegenüber dem Halogen sei beispielsweise ein Verhältnis von 2 bis 50 Mol, bevorzugt 3 bis 20 Mol, Alkylbenzol der Formel (I) pro Mol Halogen genannt.

Die Ausgangsstoffe der Formel (I) können im erfindungsgemäßen Verfahren als wasserfreie Stoffe eingesetzt werden. Es wurde jedoch gefunden, daß auch wasserhaltige Stoffe der Formel (I) eingesetzt werden können, ohne die Umsetzung im erfindungsgemäßen Verfahren zu beeinträchtigen. Als Wassergehalt sei beispielsweise ein solcher bis zur Sättigungskonzentration genannt.

Als Halogenierung der Ausgangsstoffe der Formel (I) im erfindungsgemäßen Verfahren sei die Umsetzung mit Chlor oder Brom genannt. Bevorzugt ist die Umsetzung mit Chlor.

Das Halogen wird im erfindungsgemäßen Verfahren gasförmig in der Reaktionsraum eingeleitet. Das Halogen kann ohne weiteres Verdünnungsmittel eingesetzt werden, es ist aber auch möglich, das gasförmige Halogen mit Inertgasen, beispielsweise mit Stickstoff oder mit Argon, zu verdünnen. Der Anteil des Verdünnungsmittels kann hierbei bis zu 90 %, vorzugsweise 30 bis 50 %, des eingesetzten Halogen-Inertgas-Gemisches betragen.

Die erfindungsgemäße Umsetzung wird in Gegenwart von UV-Strahlung enthaltendem Licht durchgeführt, beispielsweise unter Bestrahlung durch eine Quecksilber-Hochdruck- oder -Niederdrucklampe.

Das erfindungsgemäße Verfahren wird bei einer Temperatur von etwa 80 °C bis zum Siedepunkt des Alkylbenzols der Formel (I) durchgeführt. Bevorzugt ist der Temperaturbereich von 30 °C unter dem Siedepunkt bis zum Siedepunkt des Alkylbenzols, besonders bevorzugt der Bereich von 10 °C unter dem Siedepunkt bis zum Siedepunkt.

Das erfindungsgemäße Verfahren kann bei Normaldruck oder Überdruck, vorzugsweise bei Normaldruck, durchgeführt werden.

Die Halogenierung im erfindungsgemäßen Verfahren wird so weit durchgeführt, daß die Konzentration des durch die Monohalogenierung neugebildeten Halogenalkylbenzols in dem Reaktionsgemisch, das aus dem Reaktionsraum abgezogen wird, nicht mehr als etwa 33 Mol-%, bezogen auf die Molzahl aller im Reaktionsgemisch enthaltenen Stoffe, beträgt. Beispielsweise sei eine Konzentration von 0,1 bis 33 Mol-%, bevorzugt 2-25 Mol-%, genannt.

Das erfindungsgemäße Verfahren wird kontinuierlich durchgeführt.

Der Reaktionsraum wird zweckmäßigerweise so gestaltet, daß das flüssige Alkylbenzol und das gasförmige, gegebenenfalls mit Inertgas verdünnte Halogen gut vermischt werden.

Das Reaktionsgemisch kann durch übliche Maßnahmen, beispielsweise Destillation, Kristallisation oder Absorption, bevorzugt durch Destillation, aufgearbeitet werden. Das bei der Aufarbeitung erhältliche Endprodukt kann anschließend, beispielsweise durch fraktionierte Destillation, weiter gereinigt werden. Bei der Aufarbeitung kann das im Überschuß eingesetzte Alkylbenzol zurückgewonnen werden und wieder in das erfindungsgemäße Verfahren eingesetzt werden.

Das erfindungsgemäße Verfahren wird im allgemeinen so durchgeführt, daß man das in Bezug auf das Halogen überschüssige Alkylbenzol und das Halogen in den Reaktionsraum einbringt und dort unter der Einwirkung von UV-Strahlung enthaltendem Licht im erfindungsgemäßen Temperaturbereich umsetzt. Die Reaktionstemperatur, die Höhe des molaren Überschusses an dem Alkylbenzol, bezogen auf das Halogen, und die Verweilzeit des Reaktionsgemisches im Reaktionsraum werden hierbei in Abhängigkeit von den Ausgangsstoffen so gewählt, daß das Halogen im Reaktionsraum praktisch vollständig umgesetzt wird. Das Reaktionsgemisch wird kontinuierlich aus dem Reaktionsraum entfernt und durch Destillation aufgearbeitet.

Im erfindungsgemäßen Verfahren können Verbindungen der Formel

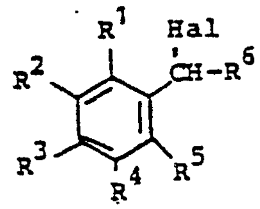

(II)

3

in der

$R^1$ bis $R^6$ die oben angegebene Bedeutung haben und Hal für Chlor oder Brom steht, hergestellt werden, beispielsweise :

Benzylchlorid, Benzylbromid, 2-Methylbenzylchlorid, 2-Methylbenzylbromid, 3-Methylbenzylchlorid, 3-Methylbenzylbromid, 4-Methylbenzylchlorid, 4-Methylbenzylbromid, 2-Chlormethyl-benzylchlorid, 3-Chlormethyl-benzylchlorid, 4-Chlormethyl-benzylchlorid, 3,4-Dimethylbenzylchlorid, 3-Methyl-4-chlormethyl-benzylchlorid, 2-Chlorbenzylchlorid, 3-Chlorbenzylchlorid, 4-Chlorbenzylchlorid, 2-Chlorbenzylbromid, 3-Chlorbenzylbromid, 4-Chlorbenzylbromid, 2,4,5-Trimethylbenzylchlorid, 2,4,5-Trimethylbenzylbromid, Tetramethylbenzylchlorid, Tetramethylbenzylbromid, Pentamethylbenzylchlorid, Pentamethylbenzylbromid, α-Methyl-benzylchlorid, α-Methyl-benzylbromid, Dichlorbenzylchlorid, Trichlorbenzylchlorid, Tetrachlorbenzylchlorid, 2-Chlorcarbonyl-benzylchlorid, 3-Chlorcarbonyl-benzylchlorid, 4-Chlorcarbonyl-benzylchlorid, 2-Fluor-benzylchlorid, 3-Fluorbenzylchlorid, 4-Fluorbenzylchlorid, 2-Fluorbenzylbromid, 3-Fluorbenzylbromid, 4-Fluorbenzylbromid, 2-Cyanobenzylchlorid, 3-Cyanobenzylchlorid, 4-Cyanobenzylchlorid, Fluor-chlor-benzylchlorid, Fluor-brom-benzylchlorid, Fluor-brombenzylbromid, Isocyanatobenzylchlorid, Chlorisocyanatobenzylchlorid, Fluor-isocyanatobenzylchlorid, Fluor-fluorcarbonyl-benzylchlorid, Fluor-cyan-benzylchlorid, Fluor-methyl-benzylchlorid, Fluor-methylbenzylbromid, Chlor-methyl-benzylchlorid, Chlor-cyan-benzylchlorid, 3-Phenoxy-benzylchlorid, 4-Fluorphenoxy-benzylchlorid, Chlorphenoxy-benzylchlorid, Phenoxy-chlor-benzylchlorid, 2-Brombenzylchlorid, 3-Brombenzylchlorid, 4-Brombenzylchlorid, 2-Brombenzylbromid, 3-Brombenzylbromid, 4-Brombenzyl-bromid, Methoxy-chlor-benzylchlorid, 3-Phenoxy-4-fluor-benzylchlorid, Phenyl-benzylchlorid, 2-Trifluormethylbenzylchlorid, 3-Trifluormethylbenzylchlorid, 4-Trifluormethylbenzylchlorid, 3-Trifluormethyl-6-chlorbenzylchlorid, 4-Trifluormethyl-5-chlor-benzylchlorid, 2-Chlor-4-trifluormethyl-benzylchlorid, 2-Chlor-3-trifluormethyl-benzylchlorid, 3-Trifluormethyl-4-chlor-benzylchlorid, 2,6-Difluorbenzylchlorid, 2,4-Difluor-benzylchlorid, 2-Chlorcarbonyloxi-benzylchlorid, 4-Trifluormethylthiobenzylchlorid, 4-Trifluormethyloxi-benzylchlorid, 3-Chlor-4-trifluormethyloxi-benzylchlorid, 4-Heptafluorisopropylbenzylchlorid, 3,5-Di-(trifluormethyl)-benzylchlorid, 4-Isocyanatobenzylchlorid, 3-Chlor-6-isocyanatobenzylchlorid, 4-Chlor-6-isocyanato-benzylchlorid, 5-Chlor-6-isocyanato-benzylchlorid, 2-Chlor-6-isocyanato-benzylchlorid, 2-Fluor-6-isocyanato-benzylchlorid, 3-Fluor-6-isocyanato-benzylchlorid, 4-Fluor-6-isocyanato-benzylchlorid, 2,3-Dichlor-6-isocyanato-benzylchlorid, 3,5-Dichlor-6-isocyanato-benzylchlorid, 2,3,4-Trichlor-6-isocyanato-benzylchlorid, 2,3,4,5-Tetrachlor-6-isocyanato-benzylchlorid, 4-Trifluormethyl-6-isocyanato-benzylchlorid, o-Nitrobenzylchlorid, m-Nitrobenzylchlorid, p-Nitrobenzylchlorid, 2-Methyl-3-Nitrobenzylchlorid, 2-Methyl-4-nitrobenzylchlorid, 2-Methyl-5-nitrobenzylchlorid, o-Chlorsulfonylbenzylchlorid, m-Chlorsulfonylbenzylchlorid, p-Chlorsulfonylbenzylchlorid, Benzalchlorid.

Die nach dem erfindungsgemäßen Verfahren herstellbaren substituierten Benzylchloride der Formel

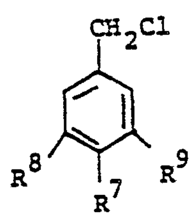

(III)

in der

$R^7$ Trifluormethoxy, Trifluormethylthio oder 2-Perfluorpropyl bedeutet, sowie für den Fall, daß $R^8$ für Chlor steht, Trifluormethyl bedeutet und weiterhin für den Fall, daß $R^8$ und $R^9$ für Trifluormethyl stehen, Wasserstoff bedeutet,

$R^8$ für Wasserstoff steht, sowie für den Fall, daß $R^7$ für Trifluormethyl oder Trifluormethoxy steht, Chlor bedeutet und weiterhin für den Fall, daß $R^7$ für Wasserstoff und $R^9$ für Trifluormethyl steht, Trifluormethyl bedeutet und

$R^9$ für Wasserstoff steht und für den Fall, daß $R^7$ für Wasserstoff und $R^8$ für Trifluormethyl stehen, Trifluormethyl bedeutet,

sind neu.

Die erfindungsgemäß durch Monohalogenierung von Alkylbenzolen in α-Stellung herstellbaren

Verbindungen können als Zwischenprodukte für die Herstellung von Pflanzenschutzmitteln, beispielsweise herbizider Wirkstoffe durch Umsetzung mit Cyankali (südafrikanische Patentanmeldung 6 801 572) oder anderer Pflanzenschutzmittel (US 1 238 522), eingesetzt werden. Sie können ferner durch Umsetzung zu den entsprechenden Sulfenamiden (analog zu DE-OS 1 941 884) in Vulkanisationsbeschleuniger umgewandelt werden, sowie als Zwischenprodukte für polymere Verbindungen, Farbstoffe (DE-AS 2 365 762) und Arzneimittel dienen. Außerdem können die erfindungsgemäß herstellbaren substituierten Benzylhalogenide zu Aldehyden umgesetzt werden (Houben-Weyl, Handbuch der Organischen Chemie, VII/1, S. 195, Georg Thieme Verlag Stuttgart 1954).

Die erfindungsgemäß durch Monohalogenierung von Alkylbenzolen in α-Stellung hergestellten Verbindungen werden in hohen Ausbeuten, im allgemeinen mehr als 90 %, meistens mehr als 94 % der theoretischen Ausbeuten, bezogen auf umgesetztes Alkylbenzol, und in besonders hoher Reinheit erhalten, so daß sie für viele Anwendungen ohne weitere Reinigung eingesetzt werden können.

Durch die erfindungsgemäße Rückführung des nicht umgesetzten Alkylbenzols (I), das vom Reaktionsgemisch nach Verlassen des Reaktionsraumes abgetrennt wird, können hohe Umsätze, im allgemeinen 80 % oder mehr des eingesetzten Alkylbenzols, ohne Unterbrechung des Verfahrens erzielt werden.

Es ist überraschend, daß im erfindungsgemäßen Verfahren auch bei hohen Umsätzen eine gleichbleibende hohe Selektivität erhalten bleibt, während im absatzweisen Verfahren nach dem Stand der Technik (Houben-Weyl, loc., cit., S. 736) beispielsweise bei der Chlorierung von Toluol bei 30 %igem Umsatz abgebrochen werden muß, um ein reines Benzylchlorid zu erhalten.

Weiterhin ist es überraschend, daß im erfindungsgemäßen Verfahren kein strenger Ausschuß von Feuchtigkeit nötig ist, wie dies nach dem Stand der Technik (Houben-Weyl, loc. cit. S. 736) gefordert wird.

## Beispiele

### A) Reaktionsapparatur

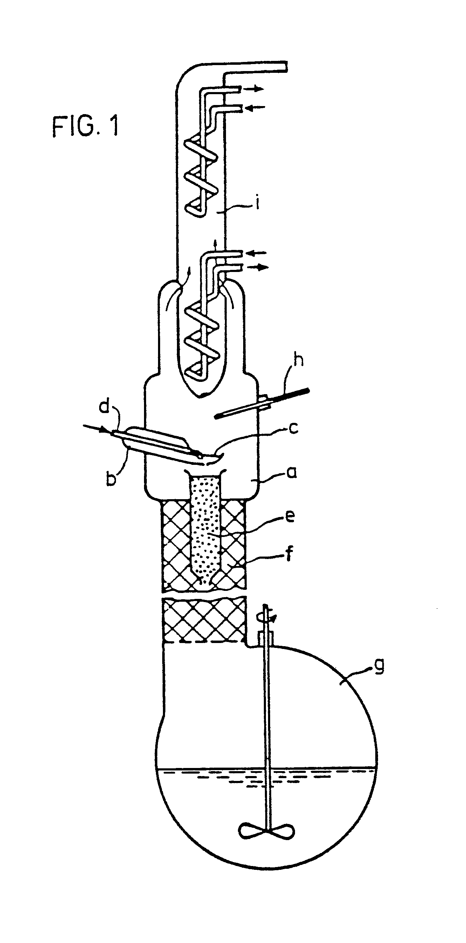

In den folgenden Beispielen wird die in Abbildung 1 dargestellte Reaktionsapparatur verwendet. Die Apparatur besteht aus einem Verdampfungsgefäß (g), das die Ausgangsverbindung enthält. Auf das Verdampfungsgefäß (g) ist eine mit Füllkörpern gefüllte Kolonne (f) aufgesetzt, in die ein leeres, sich nach unten verjüngendes Rohr (e) eingelassen ist. Oberhalb der Kolonne (f) befindet sich der Reaktionsraum (a), in den eine Vorrichtung (b) eingelassen ist, deren im Reaktionsraum befindlicher Teil zu einer Vertiefung (c) ausgebildet ist. Die Zuführung des Halogens erfolgt von außen durch den Einlaß (d) in die Vorrichtung (b) zu der Vertiefung (c), wobei gleichzeitig der Reaktionsraum mit UV-Licht bestrahlt wird. Zur Überprüfung der Reaktionstemperatur ist außerdem in dem Reaktionsraum (a) das Thermometer (h) eingelassen. Oberhalb des Reaktionsraumes ist der Kondensator (i) so angeordnet, daß die hier kondensierte flüssige Komponente in die Vertiefung (c) tropfen kann.

### B) Umsetzungen

Die Umsetzung sei an folgenden Beispielen erläutert:

### Beispiel 1

318 g m-Xylol werden in der unter A) beschriebenen Apparatur chloriert. Die Wärmezufuhr zum Verdampfungsgefäß wird dabei so geregelt, daß die vom Kondensator (i) in die Vertiefung (c) zurücklaufende Menge flüssiges Xylol etwa 900 g/Std. (etwa 8,5 Mol/Std.) beträgt. Nach dem Beginn des Rücklaufs von m-Xylol vom Kondensator wird Chlordampf in einer Menge von 33 bis 33,5 g/Std. (etwa 0,47 Mol/Std.) durch den Einlaß (d) zu der Vertiefung (c) eingeleitet. Das Reaktionsgemisch fließt durch das Loch in der Vertiefung (c) in das Rohr (e). Es enthält etwa 5 Mol-% 3-Methyl-benzylchlorid. Bei 86 % Umsatz wird die Chlorierung abgebrochen. Das Produktgemisch im Verdampfungskolben enthält laut gaschromatographischer Analyse 341 g 3-Methyl-benzylchlorid, was einer Ausbeute von 94 %, bezogen auf umgesetztes m-Xylol entspricht. Nach fraktionierter Destillation erhält man das reine 3-Methylbenzylchlorid, $Kp_{13}$ = 84 °C ; $n_D^{20}$ = 1 5354 ; Gehalt laut gaschromatographischer Analyse 98,14 %. Bei kontinuierlicher Reaktionsführung werden Chlor und m-Xylol der Apparatur oben zugeführt — während gleichzeitig fortwährend mit UV-Licht bestrahlt wird — und das Chlorierungsprodukt dem Verdampfer kontinuierlich entnommen. Die entstandene Salzsäure entweicht am Kopf der Apparatur.

Die in der Tabelle aufgeführten Beispiele wurden analog obigem Beispiel durchgeführt.

(Siehe Tabelle Seite 6 f.)

| Beispiel | Alkylbenzol | Umsetzungsgrad | Produkt nach Formel (I) und Ausbeute | |
|---|---|---|---|---|
| 2 | Toluol | 98,3 % | 94 % | 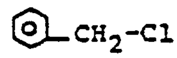 |
| 3 | o-Xylol | 89 % | 93 % | 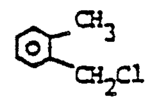 |
| 4 | p-Xylol | 91 % | 94 % | 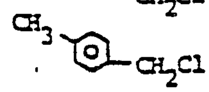 |
| 5 | Mesitylen | 81 % | 90,5 % | 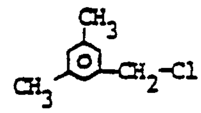 |
| 6 | 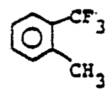 | 96 % | 98 % | 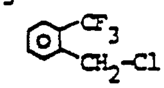 |
| 7 | 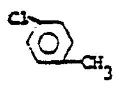 | 77 % | 98,6 % | 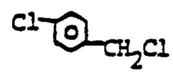 |
| 8 | 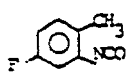 | 76,3 % | 97 % | 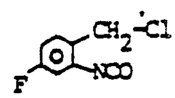 |
| 9 | 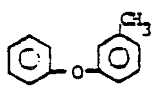 | 80 % | 90 % | 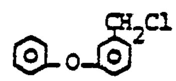 |
| 10 | 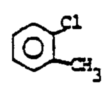 | 96 % | 99 % | 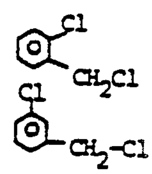 |
| 11 | 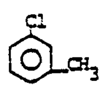 | 86 % | 99 % | |
| 12a | 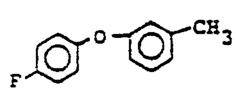 | 34 % | 90,3 % | 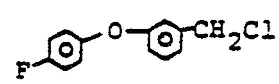 |
| 12b | 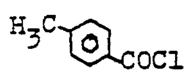 | 87 % | 96,5 % | 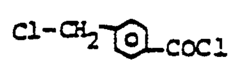 |

Nach dem Verfahren von Beispiel 1 erhält man mit gleich gutem Ergebnis folgende Verbindungen :

| | | | | | |
|---|---|---|---|---|---|
| 13 | 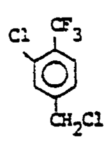 | $Kp_{12}$ | 102 °C | $n_D^{20}$ | 1.4936 |
| 14 | 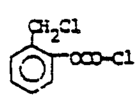 | $Kp_{12}$ | 123 °C | $n_D^{20}$ | 1.5379 |
| 15 | 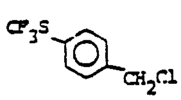 | $Kp_{15}$ | 100 °C | $n_D^{20}$ | 1.5070 |

(Fortsetzung)

| | | | | | |
|---|---|---|---|---|---|
| 16 | 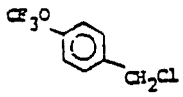 | Kp | 185 °C | $n_D^{20}$ | 1.4549 |
| 17 | 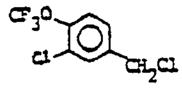 | $Kp_{15}$ | 100 °C | $n_D^{20}$ | 1.4788 |
| 18 | 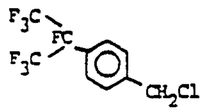 | $Kp_{50}$ | 109 °C | $n_D^{20}$ | 1.4251 |
| 19 | 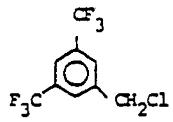 | $Kp_{16}$ | 68 °C | Schmp. | etwa 28 °C |
| 20 | 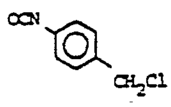 | $Kp_{10}$ | 117 °C | Schmp. | 34 °C |

Beispiele 21-24 (Vergleichsbeispiele)

Für die in der Tabelle angegebenen Alkylbenzole wurde eine Chlorierung nach Beispiel 1 mit Umsätzen von über 80 % durchgeführt und die dabei erhaltenen Nebenprodukte bestimmt. Zum Vergleich wurde eine Sumpfphasen-Chlorierung nach den Angaben in Houben-Weyl, Methoden der Organischen Chemie, Bd. V/3, S. 736, Verlag Georg Thieme, Stuttgart, 1962, durchgeführt und der Umsetzungsgrad bestimmt, bei dem die gleiche Menge an Nebenprodukten auftritt.

| Nr. | Alkylbenzol | Umsetzungsgrad (%) erfindungsgemäß | Stand der Technik | Nebenprodukte (Gew.-%) |
|---|---|---|---|---|
| 21 | Mesitylen | 81 | 40 | 7,6 |
| 22 | o-Xylol | 88,5 | 43 | 5 |
| 23 | m-Xylol | 86 | 40 | 5 |
| 24 | p-Xylol | 85 | 43 | 5 |

**Patentanspruch**

Verfahren zur Monohalogenierung von Alkylbenzolen, die in α-Stellung mindestens 1 Wasserstoffatom aufweisen und gegebenenfalls Wasser bis zur Sättigungskonzentration enthalten, in α-Stellung in flüssiger Phase im Temperaturbereich von etwa 80 °C bis zum Siedepunkt des Alkylbenzols in Gegenwart von UV-Strahlen enthaltendem Licht mit Chlor oder Brom bis zu einem Umsatz, daß die Konzentration des Monohalogenalkylbenzols im Reaktionsgemisch nicht mehr als etwa 33 Mol-%, bezogen auf die Molzahl aller im Reaktionsgemisch enthaltenden Stoffe, beträgt, dadurch gekennzeichnet, daß man die Monohalogenierung in einer Apparatur vornimmt, bei der aus einem Verdampfungsgefäß (g), in dem sich Alkylbenzol und nach Anlaufen der Reaktion Monohalogenalkylbenzol befinden, das Alkylbenzol über eine mit Füllkörpern gefüllte Kolonne (f), in die ein leeres, sich nach unten verjüngendes Rohr (e) eingelassen ist, und einen Kondensator (i) in einen Reaktionsraum (a), der sich zwischen Kolonne und Kondensator befindet und in den eine zur Zuführung des Halogens dienende Vorrichtung (b) eingelassen ist, deren im Reaktionsraum befindlicher Teil zu einer Vertiefung (c) ausgebildet ist eingebracht wird, das Reaktionsgemisch durch das vom Kondensator ständig in die Vertiefung (c) tropfende Alkylbenzol durch ein Loch in der Vertiefung (c) und über das leere Rohr (e) in die Kolonne überführt wird, wo die Trennung in Alkylbenzol, das über den Kondensator in den Reaktionsraum zurückgeführt wird und Monohalogenalkylbenzol, das in das Verdampfungsgefäß gelangt, durchgeführt wird.

**Claim**

Process for the monohalogenation of alkylbenzenes, which have at least 1 hydrogen atom in the α-position and optionally contain water up to saturation concentration, in the α-position in the liquid phase in the temperature range of about 80 °C to the boiling point of the alkylbenzene in the presence of light containing UV rays, with chlorine or bromine, up to such a conversion that the concentration of the monohalogenoalkylbenzene in the reaction mixture is no more than about 33 mol %, based on the number of mols of all of the substances contained in the reaction mixture, characterised in that the monohalogenation is carried out in an apparatus in which the alkylbenzene is passed from a vaporising vessel (g), in which alkylbenzene and, after the start of the reaction, monohalogenoalkylbenzene are present, via a column (f) filled with packings, into which an empty tube (e) which narrows towards the bottom is inserted, and a condenser (i) into a reaction space (a) which is located between the column and the condenser and into which a device (b) is inserted which is used for the introduction of the halogen and of which the section present in the reaction chamber is designed as a recess (c), the reaction mixture is passed, by means of the alkylbenzene leaving the condenser and continuously entering the recess (c) in the form of drops, through a hole in the recess (c) and via the empty tube (e) into the column, in which the separation into alkylbenzene, which is returned via the condenser into the reaction space, and monohalogenoalkylbenzene, which enters the vaporising vessel, is carried out.

**Revendication**

Procédé de monohalogénation d'alkylbenzènes qui présentent en position α au moins un atome d'hydrogène et qui contiennent éventuellement de l'eau jusqu'à la concentration de saturation, en position α en phase liquide dans un intervalle de température allant d'environ 80 °C au point d'ébullition de l'alkylbenzène, en présence de lumière contenant des rayons ultraviolets, avec du chlore ou du brome jusqu'à un degré de réaction tel que la concentration du monohalogéno-alkylbenzène dans le mélange réactionnel ne s'élève pas à plus d'environ 33 moles %, par rapport au nombre de moles de toutes les substances contenues dans le mélange réactionnel, caractérisé en ce qu'on effectue la monohalogénation dans un appareil dans lequel l'alkylbenzène est introduit depuis un récipient d'évaporation (g), dans lequel se trouvent l'alkylbenzène et, après démarrage de la réaction, un monohalogénalkylbenzène, par l'intermédiaire d'une colonne (f) remplie de corps de garnissage, dans laquelle est introduit un tube vide (e) se rétrécissant vers le bas, et d'un condenseur (i), dans une chambre de réaction (a) qui se trouve entre la colonne et le condenseur et dans laquelle est introduit un dispositif (b) servant à l'admission de l'halogène, dont la partie qui se trouve dans la chambre de réaction est réalisée en une cavité (c), le mélange réactionnel est transféré par l'alkylbenzène tombant en gouttes continuellement depuis le condenseur dans la cavité (c), par un trou ménagé dans la cavité (c) et par le tube vide (e), dans la colonne où s'effectue la séparation en alkylbenzène, qui est recyclée par le condenseur dans la chambre de réaction, et en monohalogénalkylbenzène qui arrive dans le récipient d'évaporation.

FIG. 1